Europäisches Patentamt

European Patent Office

Office européen des brevets

(11) Veröffentlichungsnummer : **0 047 450**
**B1**

(12) **EUROPÄISCHE PATENTSCHRIFT**

(45) Veröffentlichungstag der Patentschrift :
14.09.83

(51) Int. Cl.³ : **C 07 C143/30**

(21) Anmeldenummer : **81106724.8**

(22) Anmeldetag : **28.08.81**

(54) **Verfahren zur Herstellung von Naphthalin-2-sulfonsäure und Naphthalin-2.6- und -2.7-disulfonsäure.**

(30) Priorität : **04.09.80 DE 3033328**

(43) Veröffentlichungstag der Anmeldung :
**17.03.82 Patentblatt 82/11**

(45) Bekanntmachung des Hinweises auf die Patenterteilung : **14.09.83 Patentblatt 83/37**

(84) Benannte Vertragsstaaten :
**CH DE FR GB IT LI**

(56) Entgegenhaltungen :
**DE A 2 923 930**
**HOUBEN-WEYL « Methoden der organischen Chemie » 4. Auflage, Band IX 1955, GEORG THIEME VERLAG, Stuttgart Seiten 456, 476, 477**

**ULLMANNS ENCYKLOPÄDIE DER TECHNISCHEN CHEMIE 4. Auflage, Band 17 1979, VERLAG CHEMIE, Weinheim Seiten 115 bis 119**

**Houben-Weyl : Methoden der organischen Chemie, 4. Auflage, Band IX, Stuttgart (1955), Seiten 474-477, 526-528**

(73) Patentinhaber : **BAYER AG**
**Zentralbereich Patente, Marken und Lizenzen**
**D-5090 Leverkusen 1, Bayerwerk (DE)**

(72) Erfinder : **Mentzel, Werner, Dr.**
**Morgengraben 5**
**D-5000 Koeln 80 (DE)**
Erfinder : **Aumüller, Rolf, Dr.**
**Andreasstrasse 21**
**D-5090 Leverkusen 1 (DE)**
Erfinder : **Kramer, Horst-Dieter, Dr.**
**Gabriele-Münter-Strasse 6**
**D-5090 Leverkusen 1 (DE)**

# 0 047 450

Verfahren zur Herstellung von Naphthalin-2-sulfonsäure und Naphthalin-2.6- und -2.7-disulfonsäure

Die Erfindung betrifft ein Verfahren zur Herstellung von Naphthalin-2-sulfonsäure und Naphthalin-2.6- und -2.7-disulfonsäure aus Naphthalin-mono-, -di- und -tri-sulfonsäuregemische enthaltenden Lösungen.

Naphthalin-2-sulfonsäure und Naphthalin-2.6- und -2.7-disulfonsäuren sind wichtige Ausgangsprodukte für die Herstellung von Farbstoffen (s. Ullmanns Enzyklopädie der technischen Chemie, 4. Auflage, Band 17, Seiten 117-119).

Aus Houben-Weyl : Methoden der Organischen Chemie, 4. Auflage, Band IX, insbesondere Seiten 527-528 ist bekannt, daß Naphthalin-1-sulfonsäure, Naphthalin-2-sulfonsäure, Naphthalin-1,5-, -1,6-, -2,7-, -2,6-disulfonsäure und Naphthalin-1,3,5-, -1,3,6- und -1,3,7-trisulfonsäure beim Erhitzen mit wasserhaltiger Schwefelsäure einem gleichen Endzustand zustreben, der in seiner quantitativen Zusammensetzung je nach Temperatur und Schwefelsäurekonzentration verschieden ist.

Naphthalin-2-sulfonsäure wurde bislang durch Sulfonieren von Naphthalin mit Schwefelsäure wie folgt hergestellt : In einen gußeisernen Rührwerksbehälter, in dem 96 %ige Schwefelsäure vorgelegt worden war, ließ man geschmolzenes Naphthalin einlaufen und erwärmte 2 Stunden auf 163 °C. Zur hydrolytischen Spaltung der (als Nebenprodukt gebildeten) Naphthalin-1-sulfonsäure wurde etwas Wasser zugesetzt und anschließend noch 1 Stunde bei 150 °C gerührt. Nach Zugabe von 33 %iger Natronlauge und Natriumsulfit-Lösung wurden Naphthalin und Schwefeldioxid mit Dampf abgeblasen. Das beim Abkühlen auskristallisierende Natriumsalz wurde z. B. durch Zentrifugieren isoliert (s. Ullmanns Enzyklopädie der technischen Chemie, 4. Auflage, Band 17, Seite 117).

Naphthalin-2.6- und -2.7-disulfonsäure wurden bislang durch Sulfonieren von Naphthalin mit Schwefelsäuremonohydrat bei Temperaturen von 135 bis 175 °C hergestellt. Aus dem bei der Sulfonierung anfallenden, Naphthalin-2.6- und -2.7-disulfonsäure im Gewichtsverhältnis 1 : 2 enthaltenden Reaktionsgemisch wurde nach dem Verdünnen mit Wasser Naphthalin-2.6-disulfonsäure durch Zugabe von Kochsalzlösung und Natriumsulfat bei 90 °C als Dinatriumsalz ausgefällt und aus dem Filtrat Naphthalin-2.7-disulfonsäure abgeschieden (s. BIOS Final Report Nr. 1152, Seiten 4 bis 5). Nachteilig an diesem Verfahren sind unbefriedigende Ausbeuten an den gewünschten Naphthalin-β,β'-disulfonsäuren, da unter den Reaktionsbedingungen ein beträchtlicher Teil des Naphthalins zu Trisulfonsäure sulfoniert wird und der Anfall nicht verwertbarer Abfallsäure.

Es wurde nun gefunden, daß man Naphthalin-2-sulfonsäure und Naphthalin-2.6- und -2.7-disulfonsäure bzw. Gemische dieser beiden β,β'-Disulfonsäuren auch aus salzfreien, schwefelsäuren, Naphthalin-mono-, -di- und -trisulfonsäure-Gemische enthaltenden Lösungen gewinnen kann, wenn man diese Lösungen in bestimmter Weise behandelt.

Solche salzfreie schwefelsaure Naphthalinsulfonsäuregemische enthaltende Lösungen fallen z. B. bei der Herstellung von Naphthalin-1.5-disulfonsäure als Abfallsäuren an. Durch das erfindungsgemäße Verfahren werden diese Abfallsäuren, die bislang unter erheblichen Kosten vernichtet werden mußten, einer nützlichen Verwendung zugeführt.

Die salzfreien schwefelsäuren Naphthalinsulfonsäuregemische enthaltenden Lösungen enthalten keine Naphthalin-2.6-disulfonsäure und ihr Gehalt an Naphthalin-2-sulfonsäure und Naphthalin-2.7-disulfonsäure ist mit nur etwa 1 Gew.-%, bezogen auf das in der Lösung enthaltene Naphthalinsulfonsäuregemisch, vernachlässigbar gering.

Es wurde nun gefunden, daß man die in den Lösungen befindlichen Naphthalin-α-sulfonsäure und Naphthalin-α-α'- und -α-β-disulfonsäuren in Naphthalin-2-sulfonsäure bzw. Naphthalin-2.6- und -2.7-disulfonsäure überführen kann, wenn man diese Lösungen unter schonenden Bedingungen bis zu einer bestimmten Konzentration einengt und die konzentrierte Lösung einer thermischen Behandlung unterwirft. Durch diese thermische Behandlung wird eine Isomerisierung der in der eingeengten Lösung enthaltenen Naphthalin-α-monosulfonsäure und Naphthalin-α-α'- und -α,β'-disulfonsäure in Naphthalin-2-sulfonsäure bzw. Naphthalin-2.6- und -2.7-sulfonsäuren bewirkt. Die Isomerisierung verläuft über Naphthalin-2-sulfonsäure als Zwischenstufe. Zur Herstellung von Naphthalin-2-sulfonsäure wird die thermische Behandlung auf dieser Zwischenstufe beendet.

Die Erfindung betrifft daher ein Verfahren zur Herstellung von Naphthalin-2-sulfonsäure und Naphthalin-2.6- und -2.7-disulfonsäuren oder Gemischen aus Naphthalin-2.6- und -2.7-disulfonsäure aus Lösungen, die Wasser, Schwefelsäure und Gemische von Naphthalin-mono-, -di- und -tri-sulfonsäuren enthalten, das dadurch gekennzeichnet ist, daß man die Lösungen zunächst im Vakuum bei 25 bis 100, vorzugsweise 30 bis 80 mbar und Temperaturen unter 100 °C, vorzugsweise bei Temperaturen von 70 bis 90 °C einengt, bis der Wassergehalt der Lösungen auf unter 15 Gew.-%, vorzugsweise 10 bis 2 Gew.-%, gesunken ist und die so eingeengten Lösungen anschließend unter Normaldruck oder vermindertem Druck auf Temperaturen von 150 bis 200 °C, vorzugsweise 170 bis 190 °C, erhitzt, bis der Gehalt der Lösungen an Naphthalin-2-sulfonsäure auf etwa 45 Gew.-%, bezogen auf das Gesamtgewicht der in der Lösung befindlichen Naphthalinsulfonsäuren, angestiegen ist (Herstellung von Naphthalin-2-sulfonsäure) bzw. solange erhitzt, bis der Gehalt an Naphthalin-2.6- und -2.7-disulfonsäure nicht mehr zunimmt (Herstellung von Naphthalin-2.6- und -2.7-disulfonsäure).

Nach dem Abkühlen werden aus den Lösungen der Naphthalinsulfonsäuren in Schwefelsäure Naphthalin-2-sulfonsäure bzw. Naphthalin-2.6- und -2.7-disulfonsäure bzw. das Gemisch dieser beiden Naphthalin-β,β'-disulfonsäuren in an sich bekannter Weise entweder in Form der freien Säuren oder aber nach Neutralisieren der Isomerisierungslösung, in Form ihrer Alkalisalze abgetrennt.

Während der Wärmebehandlung der eingeengten Naphthalinsulfonsäurelösungen werden die Veränderungen in der Zusammensetzung des Naphthalinsulfonsäuregemisches analytisch, z. B. mittels Hochdruckflüssigkeitschromatographie, verfolgt. Bei der Herstellung von Naphthalin-2-sulfonsäure wird die Wärmebehandlung beendet, wenn der Gehalt des Naphthalinsulfonsäuregemisches an Naphthalin-2-sulfonsäure ein Maximum erreicht hat. Das Maximum liegt bei etwa 40 bis 50 Gew.-% Naphthalin-2-sulfonsäure und wird nach etwa 8- bis 12-stündigem Erwärmen erreicht. Bei weiterem Erwärmen nimmt der Gehalt des Naphthalinsulfonsäuregemisches an Naphthalin-2-sulfonsäure wieder ab und es steigt gleichzeitig der Gehalt des Naphthalinsulfonsäuregemisches an Naphthalin-2.6- und -2.7-disulfonsäure an.

Bei der Herstellung von Naphthalin-2.6- und -2.7-disulfonsäure wird die Wärmebehandlung beendet, wenn der Gehalt des Naphthalinsulfonsäuregemisches an diesen beiden Naphthalin-β,β'-disulfonsäuren das Maximum erreicht hat. Dieses Maximum liegt bei etwa 55-65 Gew.-%, bezogen auf das Gewicht des Naphthalinsulfonsäuregemisches und wird nach etwa 15 bis 30 Stunden erreicht. Nach Erreichen des maximalen Gehaltes an Naphthalin-β,β'-disulfonsäuren ändert sich die Zusammensetzung des Naphthalinsulfonsäuregemisches auch bei weiterem Erwärmen nicht mehr.

Die Wärmebehandlung kann bei Normaldruck oder unter vermindertem Druck, z. B. bei 20 bis 100 mbar, vorgenommen werden.

Für die Verwendung in dem erfindungsgemäßen Verfahren eignen sich salzfreie, schwefelsaure Lösungen von Naphthalin-mono-, -di- und -trisulfonsäuren. Solche Lösungen fallen z. B. als Abfallsäuren bei der Herstellung von Naphthalin-1.5-disulfonsäure nach den verschiedensten Verfahren an. Solche Verfahren zur Herstellung von Naphthalin-1.5-disulfonsäure sind z.B. in Ullmanns Enzyklopädie der technischen Chemie, 4. Auflage, Band 17, Seite 118 oder in der DE-OS 28 53 337 beschrieben.

Vorzugsweise werden in dem erfindungsgemäßen Verfahren salzfreie schwefelsaure Naphthalinsulfonsäuregemische enthaltende Lösungen verwendet, die etwa 30 bis 50 Gew.-% Wasser, etwa 10 bis 30 Gew.-% Schwefelsäure und etwa 15 bis 30 Gew.-% eines Gemisches aus Naphthalin-mono-, -di- und -trisulfonsäuren enthalten. Die Naphthalinsulfonsäuregemische weisen etwa folgende Zusammensetzung auf (Angaben in Gew.-% bezogen auf das Gewicht des Naphthalinsulfonsäuregemisches) :

| | |
|---|---|
| Naphthalin-1-sulfonsäure | 0,0- 0,2 |
| Naphthalin-2-sulfonsäure | 0,0- 0,2 |
| Naphthalin-2.5-disulfonsäure | 5,8- 6,4 |
| Naphthalin-1.6-disulfonsäure | 39,7-50,2 |
| Naphthalin-2.7-disulfonsäure | 1,1- 1,5 |
| Naphthalin-1.3-disulfonsäure | 9,4-10,2 |
| Naphthalin-1.7-disulfonsäure | 14,4-18,6 |
| Naphthalin-1.3.6-trisulfonsäure | 4,1-15,3 |
| Naphthalin-1.3.5-trisulfonsäure | 8,8-12,4 |
| Naphthalin-1.3.7-trisulfonsäure | 0,2- 1,5 |

Das erfindungsgemäße Verfahren kann sowohl kontinuierlich als auch diskontinuierlich durchgeführt werden. Vorzugsweise werden das Einengen der Ausgangslösungen und die Isomerisierung der eingeengten Lösungen in zwei getrennten Stufen kontinuierlich vorgenommen.

Das Einengen der erfindungsgemäß zu verwendenden Lösungen kann in üblichen Apparaturen wie Rührwerkskesseln oder ähnlichen Behältern erfolgen.

Die Wärmebehandlung kann ebenfalls in üblichen Apparaturen aus geeignetem Material, z. B. Rührwerkskesseln, Autoklaven vorgenommen werden.

Die nach der erfindungsgemäßen Behandlung anfallenden Lösungen enthalten 45 bis 60 Gew.-% Schwefelsäure und 55 bis 40 Gew.-% Naphthalinsulfonsäuregemisch.

Die isomerisierten Naphthalinsulfonsäuregemische weisen bei der Herstellung von Naphthalin-2-sulfonsäure z. B. die in der nachstehenden Tabelle 1 unter (A) angegebene Zusammensetzung, bei der Herstellung von Naphthalin-2.6- und -2.7-disulfonsäure z.B. die ebenfalls in Tabelle 1 unter (B) angegebene Zusammensetzung auf (die Angaben in Gew.-% beziehen sich auf das Gewicht des Naphthalinsulfonsäuregemisches).

(Siehe Tabelle 1 Seite 4 f.)

**0 047 450**

Tabelle 1

| | A<br>$\underline{/}$Gew.-%$\underline{7}$ | B<br>$\underline{/}$Gew.-%$\underline{7}$ |
|---|---|---|
| Naphthalin-1-sulfonsäure | 0,0 | 0,0-0,6 |
| Naphthalin-2-sulfonsäure | 62,3-65,0 | 0,0-8,0 |
| Naphthalin-1.5-disulfonsäure | 2,0-3,5 | 0,2 |
| Naphthalin-1.6-disulfonsäure | 3,0-3,5 | 6,3-3,8 |
| Naphthalin-2.6-disulfonsäure | 0,1-0,3 | 21,0-29,5 |
| Naphthalin-2.7-disulfonsäure | 1,4-2,7 | 44,6-48,6 |
| Naphthalin-1.3-disulfonsäure | 9-11,0 | 2,3-1,5 |
| Naphthalin-1.7-disulfonsäure | 8,0-8,5 | 4,9-3,6 |
| Naphthalin-1.3.6-trisulfon-<br>säure | 9,0-12,3 | 12,4-3,8 |
| Naphthalin-1.3.5-trisulfon-<br>säure | 5,0-7,0 | 5,3-0,0 |
| Naphthalin-1.3.7-trisulfon-<br>säure | 0,2-0,6 | 3,0-0,6 |

Aus den Lösungen werden Naphthalin-2-sulfonsäure bzw. Naphthalin-2.6- und -2.7-disulfonsäure in an sich bekannter Weise isoliert. So wird zur Gewinnung von Naphthalin-2-sulfonsäure die schwefelsaure Lösung der Naphthalinsulfonsäuren mit 33 %iger Natronlauge neutralisiert und die nach dem Erkalten der Lösung ausgefallene Naphthalin-2-sulfonsäure abgenutscht.

Zur Gewinnung von Naphthalin-2,6-disulfonsäure und Naphthalin-2,7-disulfonsäure wird wie folgt verfahren :

Die schwefelsaure Lösung der isomerisierten Naphthalinsulfonsäuren wird mit gesättigter Natriumchloridlösung und wäßriger 50 %iger Natronlauge gekocht. Man kühlt auf 20 °C und filtriert die Suspension über Preßbanddrehfilter, Filterpresse oder Kastennutsche.

Das erfindungsgemäße Verfahren stellt ein neues wirtschaftliches Herstellungsverfahren für Naphthalin-2-sulfonsäure und, vorzugsweise, für die Herstellung von Naphthalin-2.6- und -2.7-disulfonsäure dar. Bei der Herstellung von Naphthalin-1.5-disulfonsäure fallen je nach Verfahren 25 bis 40 Gew.-% isomere Naphthalinsulfonsäuren in Form von Abfallsäuren an. Mit Hilfe des erfindungsgemäßen Verfahrens lassen sich diese Abfallsäuren in wirtschaftlicher Weise wiederverwenden. Bei dieser Wiederverwendung ist bemerkenswert, daß die erfindungsgemäße Isomerisierung der Naphthalin-$\alpha,\alpha'$- und -$\alpha,\beta'$-disulfonsäuren durch die in der Abfallsäure ebenfalls enthaltenen Naphthalintrisulfonsäuren bei Einhaltung der erfindungsgemäßen Arbeitsbedingungen in keiner Weise beeinträchtigt wird. Die gewünschten Naphthalin-2-sulfonsäure bzw. Naphthalin-2.6- und -2.7-disulfonsäuren werden in hohen Ausbeuten und einer Reinheit von bis zu 95 % erhalten. Erst beim Überschreiten der erfindungsgemäßen Isomerisierungstemperatur tritt eine Weitersulfonierung der Naphthalin-disulfonsäuren und damit eine Verschlechterung der Ausbeuten an gewünschten Naphthalin-$\beta,\beta'$-disulfonsäuren ein.

Beispiel 1

1 100 ml einer Abfallsäure aus der Naphthalin-1.5-disulfonsäure-Herstellung (Zusammensetzung : 35, 5 Gew.-% $H_2SO_4$; 27,3 Gew.-% eines Naphthalin-mono-, -di- und -tri-sulfonsäuregemisches ; 37,2 Gew.-% Wasser) werden unter vermindertem Druck (35 mbar) und einer Temperatur von 70 bis 80 °C eingeengt, bis der Wassergehalt auf 3 Gew.-% gesunken ist. Die $H_2SO_4$-Konzentration der eingeengten Lösung beträgt 54 Gew.-%, der Gehalt an Naphthalinsulfonsäuregemisch 43 Gew.-%.

Die eingeengte Lösung wird unter Normaldruck und Rühren allmählich auf 170 °C erhitzt und 16 Stunden auf dieser Temperatur gehalten. Anschließend wird die Lösung auf Raumtemperatur abgekühlt. In der nachstehenden Tabelle 2 sind die Zusammensetzung des Naphthalinsulfonsäuregemisches vor der Wärmebehandlung (A) und die Zusammensetzung des Naphthalinsulfonsäuregemisches nach der Wärmebehandlung (B) angegeben (Gew.-% bezogen auf das Gewicht des Naphthalinsulfonsäuregemisches).

4

# 0 047 450

Tabelle 2

|  | A<br>$\sqrt{Gew.-\%}$ | B<br>$\sqrt{Gew.-\%}$ |
|---|---|---|
| Naphthalin-1-sulfonsäure | 0,0 | 0,6 |
| Naphthalin-2-sulfonsäure | 0,0 | 8,0 |
| Naphthalin-1.5-disulfonsäure | 11,2 | 0,0 |
| Naphthalin-1.6-disulfonsäure | 46,2 | 3,8 |
| Naphthalin-2.7-disulfonsäure | 1,1 | 48,6 |
| Naphthalin-2.6-disulfonsäure | 0,2 | 29,5 |
| Naphthalin-1.3-disulfonsäure | 8,2 | 1,5 |
| Naphthalin-1.7-disulfonsäure | 17,3 | 3,6 |
| Naphthalin-1.3.6-trisulfonsäure | 5,5 | 3,8 |
| Naphthalin-1.3.5-trisulfonsäure | 10,1 | 0,0 |
| Naphthalin-1.3.7-trisulfonsäure | 0,2 | 0,6 |

Aufarbeitung der Reaktionslösung

In einem 4 l Becherglas werden 600 ml gesättigte Kochsalzlösung und 400 ml Wasser vorgelegt. In diese Lösung läßt man das schwefelsaure Naphthalinsulfonsäuregemisch unter Rühren einlaufen. Dabei steigt die Temperatur in der wäßrigen Lösung auf etwa 80 °C an. Anschließend werden weitere 600 ml gesättigte Kochsalzlösung zugesetzt und 150 ml einer 50 %igen Natriumhydroxidlösung eingetropft. Die Temperatur der wäßrigen Lösung steigt dabei auf 83 °C an. Schon während des Eintropfens der Natronlauge scheidet sich das Gemisch der Naphthalin-2.6- und -2.7-disulfonsäuren als graubrauner Niederschlag ab. Die Fällung wird durch langsames Abkühlen der wäßrigen Mischung auf Raumtemperatur (durch einfaches Stehenlassen) vervollständigt. Dann wird der Niederschlag abgesaugt und gut abgepreßt. Nach viermaligem Waschen mit je 65 ml gesättigter Kochsalzlösung wird der Filterkuchen trockengesaugt. Es werden 607 g eines feuchten, entsprechend 320 g eines trockenen, Gemisches aus Naphthalin-2.6- und -2.7-disulfonsäure erhalten (= 90 % bezogen auf den Gehalt des isomerisierten Naphthalinsulfonsäuregemisches an Naphthalin-2.6- und -2.7-disulfonsäure).

Beispiel 2

In eine im wesentlichen aus einem Verdampfer, einer Füllkörperkolonne und einem Kühler bestehende Glasapparatur wird eine auf 40 °C vorgewärmte Abfallsäure (Zusammensetzung : 22,3 Gew.-% Naphthalinsulfonsäuren, 33,7 Gew.-% Schwefelsäure und 44,0 Gew.-% Wasser) mit einer Geschwindigkeit von 300 ml/Stunde kontinuierlich eingespeist. Der Verdampfer wird bei einer Temperatur von 85 °C betrieben, der Druck beträgt 50 mbar. Das Wasser wird am Kopf der Kolonne, das Konzentrat als Sumpfprodukt aus dem Verdampfer kontinuierlich abgezogen. Zusammensetzung des Sumpfproduktes : 41 Gew.-% Naphthalinsulfonsäuren, 57 Gew.-% Schwefelsäure und 2 Gew.-% Wasser. Die Zusammensetzung des Naphthalinsulfonsäuregemisches ist in der nachstehenden Tabelle 3 in Spalte (A) angegeben.

Das Sumpfprodukt wird in eine aus einem Wärmeaustauscher, einer Füllkörperkolonne und einem Kühler bestehende zweite Apparatur kontinuierlich mit einer Geschwindigkeit von 160 ml/Stunde eingespeist. Die Füllkörperkolonne wird bei einer Temperatur von 190 °C und Normaldruck betrieben. In ihr findet die eigentliche Isomerisierung statt. Das isomerisierte Naphthalinsulfonsäuregemisch tritt am Ende des Kühlers aus der Apparatur aus. Die Zusammensetzung des isomerisierten Naphthalinsulfonsäuregemisches ist in der nachstehenden Tabelle 3 in Spalte (B) angegeben.

Das isomerisierte Naphthalinsulfonsäuregemisch wird anschließend wie in Beispiel 1 beschrieben auf Naphthalin-2.6/2.7-disulfonsäure aufgearbeitet.

(Siehe Tabelle 3, Seite 6 f.)

5

Tabelle 3

| | A (Gew.-%) | B (Gew.-%) |
|---|---|---|
| Naphthalin-1-sulfonsäure | 0,0 | 0,0 |
| Naphthalin-2-sulfonsäure | 0,0 | 0,0 |
| Naphthalin-1.5-disulfonsäure | 6,4 | 0,2 |
| Naphthalin-1.6-disulfonsäure | 50,2 | 6,3 |
| Naphthalin-2.7-disulfonsäure | 1,4 | 44,6 |
| Naphthalin-2.6-disulfonsäure | 0,1 | 21,0 |
| Naphthalin-1.3-disulfonsäure | 10,2 | 2,3 |
| Naphthalin-1.7-disulfonsäure | 18,6 | 4,9 |
| Naphthalin-1.3.6-trisulfonsäure | 4,1 | 12,4 |
| Naphthalin-1.3.5-trisulfonsäure | 8,8 | 5,3 |
| Naphthalin-1.3.7-trisulfonsäure | 0,2 | 3,0 |

(Gew.-% bezogen auf das Gewicht des Naphthalinsulfonsäure-Gemisches)

**Ansprüche**

1. Verfahren zur Herstellung von Naphthalin-2-sulfonsäure aus Lösungen, die Wasser, Schwefelsäure und Gemische von Naphthalin-mono-, -di- und -tri-sulfonsäuren enthalten, dadurch gekennzeichnet, daß man die Lösungen zunächst im Vakuum bei 25 bis 100 mbar und Temperaturen unter 100 °C einengt, bis der Wassergehalt der Lösungen auf unter 15 Gew.-% gesunken ist und die so eingeengten Lösungen anschließend unter Normaldruck oder vermindertem Druck auf Temperaturen von 150 bis 200 °C erhitzt, bis der Gehalt der Lösungen an Naphthalin-2-sulfonsäure auf etwa 45 Gew.-%, bezogen auf das Gesamtgewicht der in der Lösung befindlichen Naphthalinsulfonsäuren, angestiegen ist und anschließend Naphthalin-2-sulfonsäure in an sich bekannter Weise aus dem Reaktionsgemisch isoliert.

2. Verfahren zur Herstellung von Naphthalin-2.6- und -2.7-disulfonsäure oder Gemischen aus Naphthalin-2.6- und -2.7-disulfonsäure aus Lösungen, die Wasser, Schwefelsäure und Gemische von Naphthalin-mono-, -di- und -tri-sulfonsäuren enthalten, dadurch gekennzeichnet, daß man die Lösungen zunächst im Vakuum bei 25 bis 100 mbar und Temperaturen unter 100 °C einengt, bis der Wassergehalt der Lösungen auf unter 15 Gew.-% gesunken ist und die so eingeengten Lösungen anschließend unter Normaldruck oder vermindertem Druck auf Temperaturen von 150 bis 200 °C erhitzt, bis der Gehalt der Lösungen an Naphthalin-2.6- und -2.7-disulfonsäure nicht mehr zunimmt und anschließend Naphthalin-2.6- und -2.7-disulfonsäure oder das Gemisch der beiden Disulfonsäuren in an sich bekannter Weise aus dem Reaktionsgemisch isoliert.

3. Verfahren gemäß Anspruch 1 und 2, dadurch gekennzeichnet, daß man das Einengen der Ausgangslösungen im Vakuum bei 30 bis 80 mbar vornimmt.

4. Verfahren gemäß Ansprüchen 1, 2 und 3, dadurch gekennzeichnet, daß man das Einengen der Ausgangs-Lösungen bei Temperaturen von 70 bis 90 °C vornimmt.

5. Verfahren gemäß Ansprüchen 1 bis 4, dadurch gekennzeichnet, daß man die Ausgangs-Lösungen einengt, bis ihr Wassergehalt auf 10 bis 2 Gew.-% gesunken ist.

6. Verfahren gemäß Ansprüchen 1 bis 5, dadurch gekennzeichnet, daß man die eingeengten Ausgangs-Lösungen unter Normaldruck oder vermindertem Druck auf Temperaturen von 170 bis 190 °C erhitzt.

**Claims**

1. Process for the preparation of naphthalene-2-sulphonic acid from solutions which contain water, sulphuric acid and mixtures of naphthalene monosulphonic acids, naphthalene disulphonic acids and naphthalene trisulphonic acids, characterised in that the solutions are firstly concentrated in vacuo at 25 to 100 mbar and temperatures of below 100 °C, until the water content of the solutions has fallen to below 15 % by weight and the solutions thus concentrated are then heated under normal pressure or reduced pressure to temperatures of 150 to 200 °C, until the content of naphthalene-2-sulphonic acid in the solutions has risen to about 45 % by weight, relative to the total weight of the naphthalene sulphonic acids present in the solution and then naphthalene-2-sulphonic acid is isolated from the reaction mixture in a manner which is in itself known.

2. Process for the preparation of naphthalene-2,6-disulphonic acid and naphthalene-2,7-disulphonic acid or mixtures of naphthalene-2,6-disulphonic acid and naphthalene-2,7-disulphonic acid from solutions which contain water, sulphuric acid and mixtures of naphthalene-monosulphonic acids, naphthalene disulphonic acids and naphthalene trisulphonic acids, characterised in that the solutions are firstly concentrated in vacuo at 25 to 100 mbar and temperatures of below 100 °C, until the water content of the solutions has fallen to below 15 % by weight and the solutions thus concentrated are then heated under normal pressure or reduced pressure to temperatures of 150 to 200 °C, until the content of naphthalene-2,6-disulphonic acid and naphthalene-2,7-disulphonic acid in the solutions no longer increases and then naphthalene-2,6-disulphonic acid and naphthalene-2,7-disulphonic acid or the mixture of both disulphonic acids are isolated from the reaction mixture in a manner which is in itself known.

3. Process according to Claim 1 and 2, characterised in that the starting solutions are concentrated in vacuo at 30 to 80 mbar.

4. Process according to Claims 1, 2 and 3, characterised in that the starting solutions are concentrated at temperatures of 70 to 90 °C.

5. Process according to Claims 1 to 4, characterised in that the starting solutions are concentrated until their water content has fallen to 10 to 2 % by weight.

6. Process according to Claims 1 to 5, characterised in that the concentrated starting solutions are heated to temperatures of 170 to 190 °C, under normal pressure or reduced pressure.

**Revendications**

1. Procédé de production d'acide naphtalène-2-sulfonique à partir de solutions qui contiennent de l'eau, de l'acide sulfurique et des mélanges d'acides naphtalène-mono-, -di- et -trisulfoniques, caractérisé en ce qu'on concentre les solutions tout d'abord sous vide de 25 à 100 mbars et à des températures inférieures à 100 °C jusqu'à ce que la teneur en eau des solutions se soit abaissée au-dessous de 15 % en poids, et on chauffe ensuite les solutions ainsi concentrées sous pression normale ou sous pression réduite, à des températures de 150 à 200 °C, jusqu'à ce que la teneur des solutions en acide naphthalène-2-sulfonique se soit élevée à environ 45 % en poids par rapport au poids total des acides naphthalènesulfoniques se trouvant dans la solution, puis on isole l'acide naphthalène-2-sulfonique du mélange réactionel d'une manière connue.

2. Procédé de production d'acide naphtalène-2.6- et -2.7-disulfonique ou de mélanges d'acides naphthalène-2.6- et -2.7-disulfoniques à partir de solutions qui contiennent de l'eau, de l'acide sulfurique et des mélanges d'acides naphtalène-mono-, -di- et -trisulfoniques, caractérisé en ce qu'on concentre les solutions tout d'abord sous vide de 25 à 100 mbars et à des températures inférieures à 100 °C jusqu'à ce que la teneur en eau des solutions se soit abaissée au-dessous de 15 % en poids, et on chauffe ensuite les solutions ainsi concentrées sous pression normale ou sous pression réduite, à des températures de 150 à 200 °C, jusqu'à ce que la teneur des solutions en acide naphtalène-2.6- et -2.7-disulfonique ne s'élève plus, puis on isole du mélange réactionnel, d'une manière connue, l'acide naphtalène-2.6- et -2.7-disulfonique ou le mélange des deux acides disulfoniques.

3. Procédé suivant les revendications 1 et 2, caractérisé en ce qu'on effectue la concentration des solutions de départ sous un vide de 30 à 80 mbars.

4. Procédé suivant les revendications 1, 2 et 3, caractérisé en ce qu'on effectue la concentration des solutions de départ à des températures de 70 à 90 °C.

5. Procédé suivant les revendications 1 à 4, caractérisé en ce qu'on concentre les solutions de départ jusqu'à ce que leur teneur en eau se soit abaissée à 10-2 % en poids.

6. Procédé suivant les revendications 1 à 5, caractérisé en ce qu'on chauffe les solutions de départ concentrées sous pression normale ou sous pression réduite à des températures de 170 à 190 °C.